(19) Europäisches Patentamt
European Patent Office
Office européen des brevets

(11) **EP 3 253 457 B1**

(12) **FASCICULE DE BREVET EUROPEEN**

(45) Date de publication et mention
de la délivrance du brevet:
**31.03.2021 Bulletin 2021/13**

(21) Numéro de dépôt: **16705978.1**

(22) Date de dépôt: **04.02.2016**

(51) Int Cl.:
***A61Q 19/00*** (2006.01)       ***A61Q 19/08*** (2006.01)
***A61K 9/08*** (2006.01)       ***A61K 36/534*** (2006.01)

(86) Numéro de dépôt international:
**PCT/FR2016/050238**

(87) Numéro de publication internationale:
**WO 2016/124862 (11.08.2016 Gazette 2016/32)**

(54) **UTILISATION COSMETIQUE D'UN EXTRAIT DE MENTHE POIVRÉE**

KOSMETISCHE VERWENDUNG EINES PFEFFERMINZEXTRAKTS

COSMETIC USE OF A PEPERMINT EXTRACT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **04.02.2015 FR 1550863**

(43) Date de publication de la demande:
**13.12.2017 Bulletin 2017/50**

(73) Titulaire: **LvmH Recherche
45800 Saint-Jean De Braye (FR)**

(72) Inventeurs:
• **DUMAS, Marc
45650 Saint Jean de Braye (FR)**
• **JEANNETON, Olivier
45530 Virty aux Loges (FR)**
• **JERONIMO-MONTEIRO, Valentin
45470 Trainou (FR)**

• **NIZARD, Carine
94200 Ivry sur Seine (FR)**
• **MOREAU, Marielle
78770 Marcq (FR)**

(74) Mandataire: **Cabinet Beau de Loménie
158, rue de l'Université
75340 Paris Cedex 07 (FR)**

(56) Documents cités:
**FR-A1- 2 848 851        FR-A1- 2 895 678
FR-A1- 2 962 648        FR-A1- 2 978 043
JP-A- 2006 176 436      JP-A- 2007 277 149
JP-A- 2012 236 801**

• **DATABASE WPI Week 200781 Thomson
Scientific, London, GB; AN 2007-877786 & JP
2007 277149 A (KAO CORP) 25 October 2007
(2007-10-25)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention concerne l'utilisation d'un extrait de menthe poivrée (*Mentha piperita*) dans des compositions cosmétiques, pour maintenir ou réguler le renouvellement des cellules de l'épiderme associé à une déficience de l'expression des protéines NOTCH au niveau des kératinocytes progéniteurs, pour les peaux matures et les peaux présentant des signes de sécheresse cutanée.

ETAT DE LA TECHNIQUE

On connaît de longue date l'utilisation d'extraits de menthe poivrée dans des compositions cosmétiques.

Par exemple, un extrait de *Mentha piperita* a été proposé comme actif ayant un effet apaisant pour la peau dans le document FR 2848851.

Le document FR 2895678 divulgue l'utilisation d'extraits de cellules d'au moins un végétal de la famille des menthes à titre d'agent actif dans une composition cosmétique pour traiter les désordres cutanés associés à un excès de mélanogenèse et/ou pour prévenir et/ou diminuer la synthèse et/ou la libération de mélanine, ainsi que les propriétés desdits extraits à l'égard du processus de mélanogénèse, notamment induit par un stress interne, par exemple un stress émotionnel.

JP 2007 277149 A décrit un agent promoteur de l'expression de l'involucrine, une protéine synthétisée dans le cytoplasme des kératinocytes du stratum granulosum, et de la kératinisation des cellules de la couche cornée. Il est utilisé pour lutter contre le vieillissement de la peau, la sécheresse cutanée et le photo-vieillissement.

JP 2006 176436 A décrit un inhibiteur de l'expression des cellules souches ou SCF (Stem Cell Factor) comprenant à titre de principe actif un extrait de plante choisi parmi Pureni, Hovenia dulcis, Kaphal, Gandirah, Hydrangea serrata, mélilot officinal, Zingiber officinale, Juniperus communis var. communis, Mentha X ppiperita, Rubus suavissimus, Prurus persica, Zizphus jujuba, Sasa Veitchii, isodon trichocarpus, Tilah, Phellodendron amurense, Carthamus tinctorius, Arctostaphylos uva ursi ou Nuphar japonicum.

JP 2012 236801 A décrit un agent capable de renforcer la résistance des cellules de la peau aux UV en stimulant la production de filagrine, ledit agent comprenant en tant que principe actif au moins un extrait de plante sélectionné parmi : Viscum album, Cranesbill, Shonny Haw, Mentha piperita, Cassia tora et Ziziphus jujuba.

FR 2 962 648 A1 se rapporte à une composition cosmétique ou dermatologique comprenant un extrait d'argousier, un extrait de génépi et un extrait de menthe poivrée, destinée à améliorer l'hydratation, l'éclat et la fermeté des peaux masculines, tout en assurant une protection et une purification de la peau.

FR 2 978 043 A1 décrit l'utilisation d'un extrait de menthe aquatique (Mentha aquatica) dans une composition cosmétique anti-âge.

FR 2 848 851 A1 se rapporte à un procédé d'obtention d'un principe actif ayant un effet apaisant pour la peau, à partir de Mentha piperita en solution aqueuse. Il est mentionné que cet actif limite les réactions micro-inflammatoires engendrées par les médiateurs pro-inflammatoires interleukines la et 8, prostaglandine PGE2 et histamine.

FR 2 895 678 A1 concerne un procédé de traitement non thérapeutique pour prévenir et/ou diminuer la synthèse et/ou la libération de mélanine, un excès de pigmentation cutanée ou des désordres cutanés associés à une mélanogénèse excessive, dans lequel on utilise une composition cosmétique comprenant un extrait de cellules de menthe.

Cependant aucun de ces documents ne divulgue l'action d'un extrait de menthe poivrée pour maintenir ou réguler le renouvellement des cellules de l'épiderme, en particulier le renouvellement des kératinocytes à fort potentiel de clonogénicité.

Il a en effet été découvert de manière totalement inattendue par les inventeurs de la présente invention, qu'un extrait de menthe poivrée stimule l'expression de la protéine NOTCH qui est présente dans les kératinocytes humains présentant un tel potentiel.

Parmi les kératinocytes formant l'épiderme, on distingue des cellules à fort potentiel de clonogénicité qui vont produire la plus grande quantité de cellules-filles et ainsi jouer un rôle primordial dans le renouvellement cellulaire de l'épiderme et en particulier maintenir ce renouvellement sur le long terme.

Ces kératinocytes particuliers, qu'on appellera également « cellules souches » ou « kératinocytes souches » ou « kératinocytes progéniteurs » dans la présente demande, représentent moins de 1% du nombre de cellules constituant l'épiderme et sont localisés préférentiellement au niveau de la couche basale de celui-ci, ce qui les protège plus efficacement contre des dommages à l'ADN notamment induits par l'action du rayonnement solaire, en particulier les UV-B qui sont en grande partie filtrés par les strates cellulaires supérieures de l'épiderme.

Les cellules dites d'amplification, filles des précédentes, ont un moindre potentiel de régénération que celui des « cellules-souches » définies précédemment mais participent aussi à la régénération de l'épiderme ce qui évite aux cellules souches d'être trop sollicitées à se diviser.

Les protéines NOTCH telles que les protéines NOTCH 1 et NOTCH 2, appartiennent à une famille de récepteurs transmembranaires dont l'activation se fait par une liaison directe entre le récepteur et ses ligands portés par les cellules voisines. Cette liaison provoque la libération d'une partie intracellulaire du récepteur (NICD) depuis la membrane cellulaire après clivage par la gamma-sécrétase. Le fragment NICD migre ensuite dans le noyau des kératinocytes où il va s'associer aux facteurs de transcription pour réguler un ensemble de gènes cibles (Aithal M. G. S et al J. Genet. (2013) Role of NOTCH signaling pathway in cancer and its association with DNA methylation).

Dans l'épiderme, NOTCH joue un rôle essentiel en assurant que la prolifération et la différenciation des cellules soient coordonnées pour favoriser l'homéostasie de ce tissu. Des dysfonctionnements dans le processus de signalisation de NOTCH conduit à des anomalies majeures pour l'épiderme et ses annexes causant des pertes fonctionnelles comme par exemple une barrière cutanée défectueuse ou une insuffisance de la production sébacée par la réduction du nombre de sébocytes, cellules qui produisent le sébum (Melnik BC, Acta Derm Venereol. (2014), The Potential Role of Impaired NOTCH Signalling in Atopic Dermatitis). Plusieurs pathologies résultent aussi d'un dysfonctionnement du processus de signalisation de NOTCH, l'atopie (Melnik BC, Acta Derm Venereol. (2014), The Potential Role of Impaired NOTCH Signalling in Atopic Dermatitis), le vitiligo avec la perte de mélanocytes épidermiques ou folliculaires actifs (Seleit I et al Ann Diagn Pathol. 2014 Immunohistochemical expression of aberrant NOTCH -1 signaling in vitiligo: an implication for pathogenesis), ainsi que certains cancers (Nowell C, Cold Spring Harb Perspect Med. (2013) Cutaneous NOTCH signaling in health and disease / Aithal M. G. S et al J. Genet (2013) Role of NOTCH signalling pathway in cancer and its association with DNA methylation). Ainsi un dysfonctionnement de NOTCH peut être à lui seul à l'origine de nombreuses perturbations cutanées qu'il s'agisse de la production de sébum, de la fonction barrière de la peau, et de la pigmentation

Plusieurs travaux convergent pour indiquer que la signalisation cellulaire contrôlée par NOTCH est indispensable à une bonne réparation de la peau et qu'elle serait impliquée dans la régulation de plusieurs phases du processus de cicatrisation, en particulier dans la régulation de l'angiogenèse, de la production de la matrice extracellulaire et dans l'inflammation (Lirsten A Cell. Mol. Life Sci. (2013) Cutaneous wound healing: recruiting developmental pathways for regeneration).

Il a été montré que l'expression de NOTCH diminuait dans les peaux âgées *in vivo* ainsi que, *in vitro,* dans les kératinocytes souches et leurs cellules filles dites d'amplification provenant d'individus âgés. (Palazzo E et al. J Invest Dermatol (2011), 131, S116-S117. NOTCH-1 and NOTCH-2 modulate keratinocyte stem cell viability and differentiation during skin ageing and UVB exposure).

L'inactivation de la signalisation de NOTCH bloque le cycle cellulaire et empêche les mitoses cellulaires, et ainsi le processus de régénération de l'épiderme. Cette inactivation de NOTCH provoque également une différenciation prématurée des kératinocytes souches, avec pour conséquence un tarissement du pool keratinocytaire régénératif. A l'inverse l'augmentation de l'expression de NOTCH réduit le processus de différenciation des kératinocytes (Palazzo P et al. J Invest Dermatol (2014) S2 NOTCH 1 downregulates human keratinocyte differentiation: a feedback loop with survivin). Ces travaux montrent que cette protéine est déterminante dans le maintien des populations indifférenciées de kératinocytes, progéniteurs qui assurent le renouvellement de l'épiderme. Ainsi la mise en évidence de la disparition de l'expression de NOTCH dans l'épiderme lorsque l'âge augmente et lors d'une exposition au rayonnement UVB peut être considérée comme une des causes biologiques de la perte de la régénération cellulaire cutanée au cours du vieillissement et du photo-vieillissement.

Les inventeurs ont à présent montré qu'il est possible de stimuler de façon importante l'expression de la protéine NOTCH au niveau des kératinocytes de l'épiderme, et plus précisément sur des kératinocytes possédant une capacité à se diviser, encore appelés kératinocytes progéniteurs.

Du fait de son action stimulante sur la production de NOTCH au niveau des kératinocytes progéniteurs, l'extrait de menthe poivrée est un agent particulièrement intéressant pour maintenir ou renforcer le renouvellement des cellules de l'épiderme.

On pourra mettre à profit les nouvelles propriétés de cet extrait de menthe poivrée dans le cas où l'épiderme présente un trouble de son processus régénératif.

C'est tout particulièrement le cas d'une part pour les peaux matures qui présentent une capacité de régénération réduite, et d'autre part pour les peaux déshydratées, la déshydratation pouvant affecter leur processus régénératif.

Les inventeurs ont en effet observé qu'un environnement hyperosmotique provoquant une perte d'eau cellulaire réduit la capacité des kératinocytes progéniteurs à produire des cellules filles. Ainsi l'exposition des cellules cutanées à une déshydratation réduit significativement leur capacité de régénération.

A l'appui de cette observation s'ajoute le fait que la prolifération des cellules de l'épiderme et la cicatrisation de la peau sont retardées lors d'un déficit en aquaporine-3, un canal hydrique qui en facilitant l'apport et la distribution d'eau cutanée, permet d'assurer l'équilibre hydrique de l'épiderme (M Boury-Jamot, et al. Skin Aquaporins: Function in Hydration, Wound Healing, and Skin Epidermis Homeostasis. E Beitz (ed.), Aquaporins, Handbook of Experimental Pharmacology 190, 205c Springer-Veriag Berlin Heidelberg (2009), 205-217).

Par ailleurs en raison de l'exposition de la peau à des conditions environnementales desséchantes les cellules de l'épiderme peuvent être confrontées à des situations de déshydratation. L'exposition de la peau au froid, à des nettoyages successifs peuvent perturber les lipides de la barrière cutanée qui permettent à l'eau de demeurer dans la peau, et ainsi accentuer la perte d'eau, processus qui va exposer ponctuellement les cellules cutanées à un déficit hydrique. D'autres facteurs environnementaux stressant comme l'exposition à des polluants atmosphériques oxydants (ozone) ou au rayonnement solaire, peuvent également altérer la barrière hydrique. Ainsi l'épiderme peut se retrouver fréquemment dans des situations d'exposition susceptibles de conduire à sa déshydratation.

Les inventeurs ont aussi observé qu'en retirant partiellement la barrière cutanée avec des bandes adhésives de façon à doubler la perte en eau cutanée (dite « perte insensible en eau » ou « PIE »), le processus de reconstruction de cette barrière (retour à la PIE initiale) était beaucoup plus lent lorsque la peau était sèche. Ainsi la sécheresse cutanée augmente la durée de l'exposition à la déshydratation lorsque la barrière cutanée est endommagée.

La présente invention propose donc un moyen permettant de maintenir ou réguler le renouvellement des cellules de l'épiderme associé à une déficience de l'expression des protéines NOTCH au niveau des kératinocytes progéniteurs, dans les peaux sèches et déshydratées et/ou mature.

Un autre aspect de l'invention concerne la capacité des kératinocytes souches de l'épiderme à sécréter plus fortement la neurotrophine NGF (nerve growth factor) que les autres kératinocytes (Marconi A et al. J Invest Dermatol (2003) 121, 1515-1521; Expression and Function of Neurotrophins and Their Receptors in Cultured Human Keratinocytes). Or, il a été montré que le NGF était capable de stimuler la mise en tension du collagène de type I, majoritaire dans le derme, par les fibroblastes dermiques (Palazzo E et al. J Cell Physiol. (2012) 227,1017-25, Role of neurotrophins on dermal fibroblast survival and differentiation).

Ainsi la présente invention vise également à contribuer au processus de fermeté et de tonicité du derme, en maintenant le statut de keratinocytes souches intimement lié à la forte expression de la protéine NOTCH.

Enfin les auteurs ont mis en évidence de façon inattendue un lien fort entre NOTCH et les jonctions intercellulaires communicantes appelées « jonctions GAP ». Ces jonctions communicantes permettent aux kératinocytes adjacents d'échanger entre eux les petites molécules qu'ils contiennent pour se synchroniser. Ces jonctions sont impliquées dans la régénération et l'homéostasie de l'épiderme et sont fortement altérées par le rayonnement UVA, acteur majeur du photo-vieillissement de la peau (Provost N et al. Am J Physiol Cell Physiol 284: C51-C59, 2003 Ultraviolet A radiation transiently disrupts gap junctional communication in human keratinocytes).

Les auteurs ont observé qu'en bloquant les signaux émis par la protéine NOTCH, par inhibition de la gamma-sécrétase qui en est l'orchestrateur, les échanges entre kératinocytes se produisant par les jonctions GAP et mesurés en suivant le transfert du colorant « Lucifer yellow », étaient fortement diminués. Les auteurs ont aussi mesuré que l'extrait de menthe poivrée stimule la diffusion de ce colorant entre les kératinocytes mais que cette stimulation ne se produit plus lorsque les signaux envoyés par NOTCH étaient bloqués par l'inhibiteur de gamma-sécrétase, le DAPT (N-[N-(3,5-difluorophenacetyl)-I-alanyl] -S-phenylglycinet-butyl ester). Ils montrent ainsi qu'outre l'action stimulante de l'extrait de menthe poivrée sur les échanges intercellulaires via les jonctions GAP, la stimulation s'effectue également via celle de NOTCH et de ses signaux.

Ainsi la présente invention propose-t-elle une méthode de traitement cosmétique destinée à resynchroniser les kératinocytes progéniteurs, en particulier pour lutter contre le photo-vieillissement induit par les UVA.

La mise en évidence inattendue de l'activité de l'extrait de menthe poivrée vis-à-vis de la stimulation de la production de la protéine NOTCH est particulièrement intéressante car l'expression de NOTCH dans les cellules souches cutanées diminue avec l'âge, ce qui justifie pleinement l'utilisation de l'extrait de menthe poivrée dans des compositions cosmétiques pour réaliser des soins de la peau où l'on cherche à maintenir ou à stimuler le renouvellement des cellules de l'épiderme.

BUTS DE L'INVENTION

La présente invention a ainsi pour but principal de fournir une nouvelle utilisation cosmétique d'un extrait de menthe poivrée.

La présente invention a encore pour but de fournir une méthode de soin cosmétique destinée à maintenir ou réguler le renouvellement des cellules de l'épiderme associé à une déficience de l'expression des protéines NOTCH au niveau des kératinocytes progéniteurs.

La présente invention est également particulièrement adaptée aux peaux qui présentent un déficit de production de NOTCH, les peaux dites matures, en particulier dans les zones du visage, du décolleté et des mains fortement exposée au soleil ainsi que dans les zones corporelles où la sécheresse est plus intense, par exemple au niveau des jambes et des bras et des mains mais aussi du contour de l'œil et des lèvres ainsi que dans les zones cutanées ou la perte de fermeté peut être forte, par exemple au niveau des bajoues et du menton, des paupières, du dessous des bras et des cuisses et du ventre.

La présente invention a aussi pour but de fournir une méthode de soin cosmétique destinée aux peaux sèches et/ou matures et/ou photo-vieillies et/ou déshydratées et/ou présentant des signes de relâchement.

RÉSUME DE L'INVENTION

L'invention concerne l'utilisation, dans une composition cosmétique, d'un extrait de menthe poivrée, pour maintenir ou réguler le renouvellement des cellules de l'épiderme de la peau associé à une déficience de l'expression des protéines NOTCH au niveau des kératinocytes progéniteurs, ladite composition contenant en outre un excipient cosmétiquement acceptable.

DESCRIPTION DES FIGURES

La Figure 1, donnée en référence à l'exemple 1 présente sous forme d'histogrammes, l'expression de NOTCH 1 dans des kératinocytes progéniteurs en culture exposés à un extrait de menthe poivrée.
La Figure 2, donnée également en référence à l'exemple 1, présente sous forme d'histogrammes, l'expression de NOTCH 2 dans des kératinocytes progéniteurs en culture exposés à un extrait de menthe poivrée.

DESCRIPTION DETAILLEE DE L'INVENTION

Un premier objet de l'invention concerne une utilisation cosmétique d'une composition comprenant une quantité efficace d'un extrait de menthe poivrée, encore appelée *Mentha piperita,* pour traiter une peau sèche ou une peau mature présentant des signes de vieillissement, de photo-vieillissement, de relâchement ou de sécheresse cutanée, et pour augmenter le renouvellement des cellules de l'épiderme de ladite peau, associés à une déficience de l'expression des protéines NOTCH au niveau des kératinocytes progéniteurs, ledit extrait étant un extrait des parties aériennes obtenu par une hydrolyse enzymatique en milieu aqueux.

Les cellules sont de préférence des kératinocytes localisés dans la couche basale de l'épiderme choisis dans le groupe constitué par les kératinocytes d'amplification et les kératinocytes à fort potentiel de clonogénicité, encore appelés kératinocytes souches ou kératinocytes progéniteurs.

Le renouvellement des kératinocytes est avantageusement produit par stimulation de l'expression des gènes NOTCH dans les kératinocytes, l'expression des protéines NOTCH dans lesdits kératinocytes étant déficiente.

Le renouvellement des kératinocytes est avantageusement favorisé par stimulation de l'expression du gène NOTCH dans les kératinocytes, la stimulation étant définie comme correspondant à une quantification de l'expression génique (RQ) du gène codant pour les protéines NOTCH 1 ou NOTCH 2 supérieure à 1 et significativement différente de celle-ci par un test statistique. La stimulation de l'expression du gène NOTCH est de préférence telle que la quantification de l'expression génique (RQ correspondant à la quantification du gène NOTCH par rapport à un gène invariant utilisé comme référence) est significativement supérieure ou égale à une valeur choisie dans le groupe constitué de 1,2; 1,3; 1,4; 1,5; 1,6; 1,7; 1,8; et 1,9. La quantification de l'expression génique est de préférence mesurée par PCR quantitative (qRT-PCR) en temps réel exprimée par rapport à un témoin non traité.

L'extrait de menthe poivrée peut être un extrait préparé à partir du matériel végétal issu de ladite plante, obtenue par culture *in vivo* ou *in vitro* par des techniques de culture de cellules végétales en laboratoire. Dans ce dernier mode d'obtention, on cherche à produire artificiellement des cellules végétales différenciées ou indifférenciées à partir de cellules végétales issues d'au moins un organe de la plante.

L'extrait de menthe poivrée est un extrait des parties aériennes de la menthe poivrée.

Préalablement à l'étape d'extraction, le matériel végétal peut avoir été séché et/ou broyé. Une alternative consiste à faire sécher la plante sur pied et à récolter ensuite la partie intéressante.

Selon une mise en œuvre préférée, on disperse dans l'eau le matériel végétal finement pulvérisé puis on pratique une hydrolyse enzymatique par ajout d'enzymes dans l'eau avec ajustement au pH optimal de l'enzyme.

Selon un mode de réalisation particulier, l'extrait est un extrait des feuilles, obtenu par une hydrolyse enzymatique en milieu aqueux.

L'extraction peut être menée à chaud par reflux ou bien par macération à température ambiante.

On peut avantageusement utiliser les ultrasons en cours d'extraction afin d'améliorer le rendement massique de ladite extraction.

Le procédé d'extraction peut également comprendre au moins une étape de séparation de la phase solvant et du matériel végétal épuisé par décantation, ultrafiltration ou centrifugation, de décoloration et/ou de purification et/ou de délipidation, par exemple sous la forme d'un traitement de l'extrait par une solution d'au moins un solvant polaire en présence de particules de charbon actif et/ou par un traitement de l'extrait à l'aide d'un solvant apolaire acceptable d'un point de vue cosmétique ou dermatologique, notamment un alcane en comprenant 6 ou 7 atomes de carbone ou le $CO_2$ à l'état supercritique.

Le procédé d'extraction peut en outre être complété par une étape d'élimination partielle ou totale des solvants d'extraction de façon à concentrer l'extrait.

Dans le premier cas, on concentre généralement l'extrait jusqu'à obtenir un concentré dépourvu d'une quantité significative de solvants organiques, dans le second cas on obtient un résidu sec.

De façon alternative, le produit de l'étape d'extraction peut être, atomisé et/ou lyophilisé pour se présenter sous la forme d'une poudre.

L'extrait de menthe poivrée peut être utilisé dans une composition cosmétique sous la forme d'une poudre ou bien sous la forme d'une solution ou d'une suspension de ladite poudre dans au moins un solvant cosmétiquement acceptable, qui peut être identique ou différent de celui ayant servi à l'extraction.

A titre d'exemple d'extrait utilisable dans le cadre de la présente invention, on peut utiliser un extrait préparé par la méthode décrite dans le document FR 2848851.

On pourra également utiliser des extraits de menthe poivrée disponibles dans le commerce. On cite à titre d'exemple un extrait de feuilles de menthe poivrée avec du propylène glycol commercialisé par la société ALBAN MULLER, ou un extrait de feuilles de menthe poivrée commercialisé sous la dénomination CALMISKIN® par la société SILAB.

Sauf mention contraire, les quantités d'extrait de menthe sont indiquées en matière sèche. Par "matière sèche", on désigne la quantité de matière résiduelle après dessiccation de l'extrait, telle qu'elle peut être mesurée par toute méthode connue de l'homme de l'art. La mesure peut par exemple être effectuée selon le protocole décrit dans la demande de brevet FR 2848851.

L'extrait de menthe poivrée est présent en une quantité efficace pour maintenir ou réguler le renouvellement des cellules de l'épiderme, et notamment pour stimuler efficacement la production de NOTCH au niveau des cellules localisées au niveau de la couche basale de l'épiderme, telles que les cellules à fort potentiel de clonogénicité et/ou les cellules d'amplification.

Ainsi, la composition cosmétique utilisée dans le cadre de l'invention peut comprendre de 0,005 à 0,5% en poids de matière sèche de l'extrait par rapport au poids de la composition, avantageusement entre 0,02% et 0,2% en poids de matière sèche par rapport au poids total de la composition.

Outre un extrait de menthe poivrée tel que décrit précédemment, les compositions selon l'invention peuvent comprendre un ou plusieurs autres agents actifs cosmétiquement acceptables présentant des effets cosmétiques similaires et/ou complémentaires de l'extrait de menthe poivrée, des excipients et/ou support de formulation sélectionnés plus particulièrement au regard du mode d'administration considéré pour la composition.

On pourra associer en particulier à l'extrait de menthe poivrée, un actif qui agit sur l'expression de NOTCH dans les kératinocytes humains normaux choisi parmi un extrait aqueux de bourgeons de Hêtre (*Fagus Sylvatica*), par exemple celui commercialisé sous le nom de Gatuline® RP par la société Gatefossé ou encore un extrait aqueux de fleurs de *Bellis perennis,* par exemple celui commercialisé sous le nom de Belides® par la Société CLR.

L'extrait de menthe poivrée pourra avantageusement être associé à une quantité efficace d'extraits stimulant ou favorisant la formation de la survivine, protéine fortement exprimée dans les kératinocytes progéniteurs et possèdant un rôle protecteur vis-à-vis de l'apotose cellulaire, comme par exemple, l'extrait de *Coleus forskolii,* de *Limnophila conferta* et de *Lepechinia caulescens* ou encore un extrait *d'Oryza sativa* comme le Survixyl RZ™ d'Ashland stimulant un complexe protéique appelé CPC (pour chromosomal passenger complex) contenant la survivine.

On pourra associer en particulier à l'extrait de menthe poivrée, un ou plusieurs extraits de plantes connus pour ralentir ou prévenir l'apparition des signes de sécheresse cutanée par une action humectante apportée par des petites molécules hydrophiles, une action stimulante de la formation d'acide hyaluronique comme avec l'extrait de feuille *d'Alpinia galanga* ou inhibant sa dégradation, une action stimulant la formation des constituants des jonctions serrées de l'épiderme ou « tight junctions » pour limiter la perte d'eau intercellulaire comme avec un extrait de racines *d'Ophiopogon japonicus,* une action stimulante de la formation du récepteur de l'acide hyaluronique ou CD44 comme avec l'extrait de *Viola tricolor,* une action stimulante de la formation des jonction cohésives de l'épiderme, desmosomes et cornéo-desmosomes comme avec l'extrait *d'Arolea lavanda,* une action stimulante de la formation des lipides épidermiques en particulier des céramides, des acides gras et du cholestérol qui participent à la fonction de barrière à l'eau comme avec un extrait de millet, une action stimulante de la synthèse du sébum en particulier de squalène et des triglycérides, ou à l'inverse une action inhibitrice de la 5-alpha-réductase comme l'extrait de graines de *Linum usitatissimum,* ou encore une action semi-occlusive à la surface de la peau par une formule filmogène.

L'extrait de menthe poivrée peut également être associé à une ou plusieurs molécules et/ou un ou plusieurs extraits végétaux présentant des propriétés hydratantes, tels que les glycols, en particulier le glycérol, ou des polyols naturels, des céramides naturels ou de synthèse, l'urée, l'acide hyaluronique, l'acide lactique.

Il est particulièrement intéressant d'associer à l'extrait de menthe poivrée, un agent actif ou des agents actifs qui, utilisés seuls ou en combinaisons, augmentent l'expression des aquaporines au niveau des cellules de la peau, tel qu'un extrait *d'Ajuga turkestanica,* un extrait de *Paeonia suffrutica,* un extrait de roses, de *kniphophia uvaria,* d'*Helianthus annuus, d'Oriza sativa,* de *Malva sylvestris,* de *Sanguisorba officinalis,* de *Glycine max,* de *Saccharomyces cerevisiae,* d'*Onopordum acanthrium,* de *Zea mays,* de *Filipendula ulmaria,* de *Salix alba,* de *Rhodophyccea, de Centella asiatica,* et d'acide ascorbique-2-glucoside.

L'extrait de menthe poivrée peut également être avantageusement associé, dans des compositions cosmétiques, avec au moins un extrait d'une ou plusieurs plantes appartenant à la famille des orchidées (*Orchidaceae*), en particulier un extrait d'au moins une orchidée du genre Vanda tel que l'orchidée *Vanda coerulea* qui présente une action sur les aquaporines des cellules de l'épiderme ou *Dendrobium* ou bien encore *Phalaenopsis.*

Il peut être également particulièrement intéressant d'associer à l'extrait de menthe poivrée, une ou plusieurs molécules ou extraits de plantes présentant des propriétés anti-oxydantes tels que par exemple les polyphénols, l'acide tannique, l'épigallocathéchine et les extraits naturels en contenant, l'épigallocatéchine-3-gallate, les anthocyanes, les carotènes, les extraits de romarin, les extraits de feuilles d'olivier, le thé vert, le resvératrol et ses dérivés, le pycnogénol, l'ergothionéine, la N-acétylcystéine, la biotine, les chélatants, l'idébénone, des extraits végétaux comme le Pronalen Bioprotect TM de la société Provital, , le co-enzyme Q10, les bioflavonoides, les SOD (superoxyde dismutase), le phytantriol, les lignanes, la mélatonine, les pidolates, un ester de tocophérol ou un de ses sels, l'acide ascorbique et ses sels, le pyrrolidone carboxylate d'arginine, les dérivés séléniés, le gluthation, et les différentes isoformes de tocotriénol et de vitamine E dont la vitamine E phosphate et l'ergothionéine.

L'extrait de menthe poivrée peut avantageusement être associé à des filtres chimiques ou physiques contre les rayonnements solaires de type UVA et UVB tels que les filtres de synthèse associés ou non à des filtres constitués de particules capables d'atténuer les effets de ce rayonnement sur la peau, et/ou des particules capables de réfléchie la lumière du soleil.

Le filtre UV peut être choisi parmi les filtres UV minéraux, les filtres UV organiques hydrophiles, les filtres UV organiques liposolubles.

Par "filtre UV organique hydrophile", on entend tout composé organique absorbant un rayonnement ultra-violet (UV) dans la gamme de longueurs d'onde allant de 280 nm à 400 nm qui peut être dissous dans la phase aqueuse de la composition, ou qui peut y être dispersé sous forme colloïdale ou sous forme micellaire.

Par "filtre UV organique liposoluble", on entend tout composé organique absorbant un rayonnement UV dans la gamme de longueurs d'onde allant de 280 nm à 400 nm qui peut être dissous à l'état moléculaire dans une huile, ou être dispersé dans une huile sous forme colloïdale ou sous forme micellaire.

La composition de l'invention comprend avantageusement au moins un filtre UV minéral choisi parmi des pigments d'oxydes métalliques.

Ces pigments sont avantageusement des oxydes de titane (amorphe ou cristallisé sous forme rutile et/ou anatase), de fer, de zinc, de zirconium ou de cérium.

Les particules de pigments d'oxydes métalliques ont une taille moyenne (D50) généralement comprises entre 5 nm et 100 nm, de préférence entre 10 nm et 50 nm.

Les pigments peuvent avoir été traités en surface, c'est-à-dire avoir subi un ou plusieurs traitements de surface de nature chimique, électronique, mécanochimique et/ou mécanique avec des composés tels que des oxydes de silicium, des oxydes métalliques tels que l'oxyde de cérium, de l'alumine, de la silice, des composés de l'aluminium, des composés du silicium, ou leurs mélanges des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones choisies dans le groupe contenant les alkyl silanes, les polydialkylsiloxanes, et les polyalkylhydrogénosiloxanes, des protéines (collagène, élastine), des alcanolamines ou de l'hexamétaphosphate de sodium.

Parmi les filtres UV hydrophiles, on peut utiliser les filtres UV suivants désignés ci-dessous par leur nom INCI ou leur nom chimique:

- l'acide téréphthalylidène dicamphosulfonique (Nom INCI : Terephthalylidène Dicamphre Acide Sulfonic Acid) commercialisé sous le nom MEXORYL® SX par CHIMEX,
- les dérivés du bis-benzoazolyle tels que décrits dans les brevets EP 669 323 et US 2,463,264 et plus particulièrement le composé Disodium Phenyl Dibenzimidazole Tetra-sulfonate vendu sous le nom commercial commercial NEO HELIOPAN® AP par Haarmann et Reimer,
- l'acide p-aminobenzoïque (Nom INCI : PABA) et ses dérivés, tels que le 1-(4-aminobenzoate)-1,2,3-propanetriol (nom INCI : Glyceryl PABA) et le PEG-25 PABA vendu sous le nom UVINUL® P25 par BASF,
- l'acide 2-phénylbenzimidazole-5-sulfonique (nom INCI : Phenylbenzimidazole Sulfonic Acid) vendu notamment sous le nom commercial EUSOLEX® 232 par MERCK,
- le salicylate de triéthanolamine,
- le 3-(4'-sulfobenzylidène) camphre (nom INCI : Benzylidene camphor sulfonic acid) commercialisé sous le nom MEXORYL® SL par CHIMEX,
- le Méthylène bis-Benzotriazolyl Tétraméthylbutylphénol (Dénomination USAN : BISOCTRIZOLE) vendu sous la référence Tinosorb® M, ou MIXXIM® BB/100 par FAIRMOUNT CHEMICAL;
- le 3-(4'-triméthylammonium benzylidène)-1-bornan-2-one méthyl sulfate (Nom INCI : Camphor Benzalkonium Methosulfate) commercialisé sous le nom "MEXORYL SO" par CHIMEX,
- la Benzophénone-4 vendu sous le nom commercial UVINUL® MS40.

On peut également utiliser comme filtre organique UV hydrophile, des molécules organiques filtrant les UV qui sont de nature lipophile (dissoutes ou dispersées dans un liquide non aqueux) qui ont été rendues hydrophiles par adsorption sur un support hydrophile de faible granulométrie, comme des particules de polymère. On pourra citer par exemple la bis-éthylhexyloxyphénol méthoxyphényl triazine, qui est un filtre UV lipophile adsorbé sur des particules de polyméthacrylate de méthyle (PMMA). Le filtre UV organique hydrophile peut donc être une molécule organique lipophile filtrant les UV, adsorbée ou absorbée sur un support hydrophile, lequel peut ne pas filtrer les UV, tel qu'un polymère organique.

Les filtres UV organiques liposolubles peuvent notamment être choisis parmi différentes familles de composés chimiques. On peut notamment citer les dérivés de l'acide para-aminobenzoïque, les dérivés salicyliques, les dérivés cinnamiques, les aminobenzophénones, les dérivés anthraniliques, les dérivés du dibenzoylméthane, les dérivés de [beta],[beta]'-diphénylacrylate, les dérivés du benzylidène camphre, les dérivés du phényl benzotriazole, les dérivés triazine, les bis-résorcinyl triazines, les dérivés d'imidazolines, les dérivés du benzalmalonate, les dérivés de 4,4-diarylbutadiène, les dérivés de benzoxazole, les mérocyanines et leurs mélanges.

La teneur en filtre UV dans la composition varie avantageusement de 0,5 à 40 % en poids, de préférence de 5 à 30 % en poids, et encore plus préférentiellement de 10 à 20 % en poids, par rapport au poids total de la composition.

Les compositions peuvent se présenter sous toutes les formes galéniques normalement utilisées selon le mode d'application ou d'administration préféré. Ces compositions sont préparées selon les méthodes usuelles.

De façon connue, les compositions peuvent contenir des adjuvants habituels dans le domaine cosmétique, tels que les matières grasses, les émulsionnants, les gélifiants hydrophiles ou lipophiles, les actifs hydrophiles ou lipophiles, les conservateurs, les antioxydants, les parfums, les charges, et les matières colorantes.

Les compositions sont de préférence formulées pour une application sur la peau. Elles présentent un effet particulièrement recherché pour maintenir ou réguler le renouvellement des cellules de l'épiderme associé à une déficience de l'expression des protéines NOTCH au niveau des kératinocytes progéniteurs, lorsqu'on applique ladite composition sur la peau du visage ou du corps.

Les compositions sont particulièrement formulées pour convenir aux peaux présentant des signes de sécheresse ou aux peaux matures et/ou sèches. La présente invention est particulièrement adaptée aux peaux qui présentent un trouble de son processus régénératif - par exemple un déficit de production de NOTCH - telles que les peaux dites sèches ou matures.

Les compositions sont appliquée sur la peau, en particulier dans les zones du visage, et plus particulièrement du contour de l'œil, du décolleté et des mains fortement exposée au soleil, ainsi que dans les zones corporelles où la sécheresse est plus intense, par exemple au niveau des jambes et des bras et des mains, mais aussi des lèvres ainsi que dans les zones cutanées ou la perte de fermeté peut être forte, par exemple au niveau des bajoues et du menton, des paupières, du dessous des bras et des cuisses et du ventre.

La composition cosmétique peut être par exemple sous la forme de solutions aqueuses, hydroalcooliques ou huileuses, de dispersions du type des solutions ou dispersions du type lotion ou sérum, d'émulsions eau-dans-huile ou huile-dans-eau ou comprenant une phase à base de glycérol ou de silicone, de consistance liquide ou semi-liquide du type lait, de suspensions ou émulsions du type crème, de gel aqueux (hydrogels) ou anhydre, de microémulsions, de microcapsules ou microparticules dispersées dans une phase continue liquide ou semi-liquide, ou de dispersions vésiculaires de type ionique et/ou non ionique.

Les compositions peuvent se présenter par exemple sous la forme de crèmes de soin, de gels de soin, de masques, de produits de maquillage tels que des mascaras ou des rouges-à-lèvres, ou de démaquillants, ou bien encore sous la forme d'un masque, d'un stick, ou encore d'un patch.

Lorsque la composition est une émulsion, elle comprend une phase grasse comprenant des huiles, ainsi que des émulsionnants et co-émulsionnants choisis parmi ceux classiquement utilisés dans le domaine cosmétique.

Parmi les huiles utilisables en cosmétique, on peut citer les huiles minérales telles que le polyisobutène hydrogéné et l'huile de vaseline, les huiles végétales issues du beurre de karité, du tournesol ou encore d'amandes d'abricot, les huiles animales, les huiles de synthèse notamment l'huile de Purcellin, le myristate d'isopropyle et le palmitate d'éthyl hexyle, et les huiles fluorées comme par exemple les perfluoropolyéthers. On peut aussi utiliser des alcools gras, des acides gras comme par exemple l'acide stéarique, des cires notamment de paraffine, carnauba ou la cire d'abeilles. On peut aussi utiliser les huiles siliconées et par exemple les cyclométhicone et diméthicone, les cires, résines et gommes siliconées.

Comme émulsionnants, on peut citer par exemple le stéarate de glycérol, le polysorbate 60, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (200E).

Comme gélifiants hydrophiles, on peut citer les polymères carboxyliques, les copolymères acryliques, les polyacrylamides, les polysaccharides comme les dérivés cellulosiques, les gommes naturelles et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

Selon leur nature, ces excipients sont introduits dans la phase grasse, dans la phase aqueuse et/ou dans les vésicules lipidiques.

La présente invention concerne, comme exposé précédemment, une utilisation d'un extrait de menthe poivrée en tant qu'agent cosmétique destiné à maintenir ou réguler le renouvellement des cellules de l'épiderme de la peau associé à une déficience de l'expression des protéines NOTCH au niveau des kératinocytes progéniteurs, en particulier pour les peaux matures et/ou sèches. Par « peau mature », on entend la peau d'une personne ayant plus de 35 ans, plus de 40 ans, voir plus de 45 ans.

L'invention a encore pour objet une méthode de soin cosmétique de la peau pour traiter une peau sèche ou une peau mature et pour maintenir ou réguler le renouvellement des cellules de l'épiderme de ladite peau présentant des signes de vieillissement, de photo-vieillissement, de relâchement ou de sécheresse cutanée associés à une déficience de l'expression des protéines NOTCH au niveau des kératinocytes progéniteurs, ladite méthode comprenant l'application sur au moins une partie du visage ou du corps d'une quantité efficace d'un extrait de menthe poivrée, ledit extrait étant un extrait des parties aériennes obtenu par une hydrolyse enzymatique en milieu aqueux.

La méthode cosmétique de l'invention peut être mise en œuvre par application topique, journalière ou plusieurs fois par jour, de la composition décrite précédemment.

Les caractéristiques qui ont été décrites en rapport avec le premier objet de l'invention relatif à l'utilisation d'un extrait de menthe poivré pour maintenir ou réguler le renouvellement de l'épiderme, s'appliquent aux caractéristiques de la méthode de l'invention, qui constitue un deuxième objet de l'invention.

Comme cela ressort clairement des exemples ci-après, la demanderesse a mis en évidence l'intérêt d'un extrait de menthe poivrée pour stimuler l'expression de la protéine NOTCH, en particulier dans les cellules à fort potentiel de clonogénicité ou cellules-souches.

D'autres buts, caractéristiques et avantages de l'invention apparaîtront clairement à la lumière de la description explicative qui va suivre faite en référence à des exemples de tests mettant en évidence les propriétés susmentionnées pour l'extrait de menthe poivrée, et des exemples de composition cosmétique utilisant cet agent, donnés simplement à titre d'illustration.

Dans les exemples, tous les pourcentages sont donnés en poids, la température est en degrés Celsius, la pression est la pression atmosphérique, sauf indication contraire.

### Exemple 1: Evaluation des effets d'un extrait de menthe poivrée sur l'expression des gènes codant pour la protéine NOTCH

L'étude a été réalisée à partir de clones de kératinocytes selon la méthode développée par Barrandon et Green (Barrandon Y and Green H. ; Proc Natl Acad Sci USA 1987, 84 : 2302-2306) qui permet d'analyser et de distinguer des kératinocytes possédant des fortes capacités à former des clones (également dénommés « kératinocytes-souches » ou « cellules-souches » ou « progéniteurs »), ou des capacités intermédiaires ou faibles (cellules filles dites d'amplification).

L'objectif de cette étude était d'évaluer l'effet de l'extrait de menthe poivrée sur des cellules souches en culture.

Plusieurs extraits dont CALMISKIN® ont été testés aux doses reportées dans le tableau suivant. Les doses sont exprimées en pourcentage en poids de la solution commerciale dans le milieu de culture cellulaire.

| Nom produit | Doses testées |
|---|---|
| Calmiskin GR® | 1.5%; 0.75%; 0.375%; 0.18%; 0.09% |
| Gatuline RP® | 5%; 2.5%; 1.25%; 0.625% |
| Belides® | 0.625% ; 0.312% ; 0.156% ; 0.078% |

### I. Traitement des cellules KHN

On utilise des Kératinocytes Humains Normaux (KHN) issus d'une plastie abdominale d'un donneur féminin de 26 ans.

Les cellules ont été fournies à confluence dans le milieu Epilife à P6 avec une densité d'ensemencement de 125000 cellules par puits, dans des plaques 12 puits. Les cellules sont ensuite traitées avec les différents ingrédients actifs préparés extemporanément aux concentrations finales d'utilisation dans du milieu Epilife sans complément. Après 24 heures de traitement, les cellules sont récupérées afin d'en extraire les ARN totaux.

## II. RT-PCR quantitative en temps réel

### II.1 Obtention des ARN totaux à l'aide du MicroLabSTAR (HAMILTON)

Le milieu de culture des cellules est éliminé, et 250 μL de tampon de lyse RLT (fourni dans le kit Nucleospin RNA, Réf. 740709.4, Macherey-Nagel) sont ajoutés. Les cellules sont raclées à l'aide d'un Cell Scraper puis le lysat cellulaire, récupéré dans une plaque deepwell 1,2 mL (fourni dans le kit Nucleospin RNA). Les ARN totaux sont ensuite extraits. Les solutions d'ARN totaux obtenues sont dosées à l'aide d'un lecteur de microplaques, le spectrostarNANO (BMG Labtech) couplé au MicrolabSTAR.

Cet appareil est relié à l'ordinateur pilotant la plateforme Robotique et possède le logiciel spécifique d'analyse des résultats. La technique nécessite une micro-plaque de 384 puits (384 μclear plate black LoBase ref :788876 Greiner), un contrôle positif (RNA 250 ref : AM7155 Applied) permettant de valider les pipetages réalisés par le robot ainsi que les valeurs générées par le lecteur spectrostarNANO.

### II.2 Synthèse des ADN complémentaires

Le kit de reverse transcription (RT) utilisé est le High Capacity Reverse Transcription Kit (Réf. 4368813, Life Technologies-Applied), utilisé selon le protocole fourni. 500 ng d'ARN totaux sont dilués dans de l'eau pour un volume final de 25μL. Ils sont ensuite incubés pendant 10 minutes à 25°C puis 2 heures à 37°C en présence de 25 μL de mélange réactionnel de High Capacity Reverse Transcription Kit 2X préalablement préparé comme indiqué ci-dessous. Les différentes manipulations sont réalisées par la plateforme MicrolabSTAR et les différentes incubations sont faites au sein du TRobot (Biométra).

Le mélange réactionnel High Capacity Reverse Transcription Kit 2X pour 1 réaction présente la composition suivante :

| Réactifs | volume |
|---|---|
| RT buffer | 5 μL |
| dNTP buffer | 2 μL |
| Random primer | 5 μL |
| RNAse out | 0,5 μL |
| RT | 2,5 μL |
| H2O | 10 μL |

### II.3 PCR quantitative en temps réel

L'effet des traitements est évalué par PCR quantitative en temps réel avec le bloc 96 puits fast du 7900HT et les réactifs de chez Life Technologies-Applied. Les amorces TaqMan gene expression assay utilisées sont :

- Gène de ménage : bêta-2-microglobuline (β2M): Hs99999907_m1
- Gène cible NOTCH 1: Hs00413187
- Gène cible NOTCH 2: Hs00225747

Mélange réactionnel pour 1 réaction :
TaqMan Fast universal PCR master mix (2X) (Réf 4352043, 10 μL) TaqMan gene expression assay 20X (Hs00000000_m1), 1 μL H2O, 4 μL

Les 15 μL du mélange sont placés dans les puits d'une plaque 96 puits spécialement conçue pour l'appareil 7900HT (ref 4346906) 5 μL d'eau sont ajoutés (pour le blanc) ou 5 μL de dilutions successives d'ADNc (pour la gamme) ou 5 μL d'échantillons dans les puits correspondants. La totalité des étapes sont réalisées par le MicrolabSTAR.

### II.4. Analyse des résultats

La PCR quantitative en temps réel peut être exploitée si son efficacité est comprise entre 90% et 110%. Cette efficacité est évaluée par la mise en place d'une gamme étalon à partir de dilutions en série d'ADNc extraits des cellules appropriées. La valeur de pente obtenu traduit l'efficacité d'amplification ; une PCR est efficace à 100% lorsque la règle du 2n (n=nombre de cycles, à savoir 40) est respectée et que la valeur de pente est de -3,32. Pour chaque échantillon, le nombre de cycles auquel apparaît le signal est déterminé par le logiciel SDS 2.3, grâce à la droite de calibration établie avec la gamme étalon, la concentration en nombre de copies de transcrit peut ainsi être calculée. Pour un même essai, les niveaux d'expression des transcrits d'intérêt obtenus sont normalisés par rapport à la valeur obtenue pour le gène de ménage Béta-2-microglobuline. Ce gène dont l'expression est constitutive et invariante permet de s'affranchir de toute variation de quantités entre essais et notamment due à des efficacités de l'étape de Reverse Transcription différentes. La quantification de l'activité transcriptionnelle est déterminée par la différence de Ct (Cycle threshold) du gène cible et du gène de ménage. Il s'agit de la quantification relative qui est obtenue par le calcul suivant où le non traité est égal à 1.

$$RQ = 2\text{-}\Delta\Delta Ct = 2\text{-}(Ct \text{ gène cible traité} - Ct \text{ gène cible non traité}) / 2\text{-}(Ct \text{ gène de ménage traité} - Ct \text{ gène de ménage non traité})$$

Afin d'évaluer des variations d'activité transcriptionnelle statistiquement significative, le test t de Student a été utilisé. Chaque condition est réalisée en triplicate (3 non traités et 3 traités dans les mêmes conditions). Le test-F de Fischer est tout d'abord appliqué en comparant les deux matrices de données. Les variations transcriptionnelle significatives seront celles qui ont un test t de Student inférieur à alpha = 0,05. Les données présentées dans la partie Résultats sont les ratios des quantités obtenues dans les échantillons traités versus les échantillons non traités le tout rapporté au gène de ménage Beta-2-microglobuline.

Le test statistique de Student a été utilisé pour comparer l'ingrédient actif en fonction de son témoin solvant. Les résultats ont été considérés comme significatifs pour $p < 0.05$ (*).

### Résultats

On observe une stimulation de l'expression du gène NOTCH par la solution d'extrait de menthe poivrée CALMISKIN®.

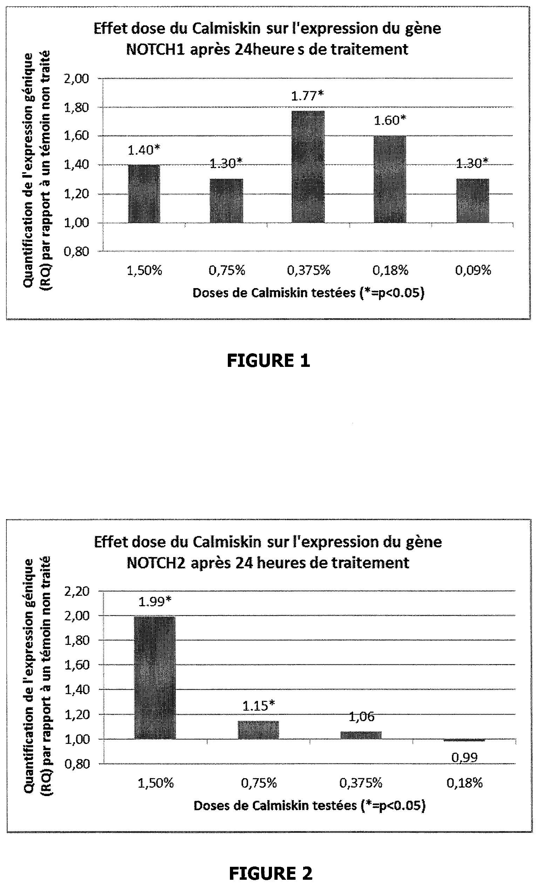

Les résultats sont présentés sur les Figures 1 et 2.

On note que le gène NOTCH 1 est répondant à des concentrations d'extrait plus faibles que NOTCH 2.

### Conclusions

L'extrait de menthe poivrée stimule l'expression des gènes NOTCH 1 et 2.

Les résultats montrent qu'un extrait de menthe poivrée stimule l'expression de la protéine NOTCH 1 dans la gamme de concentrations de 0.09% à 1.5% mais aussi de NOTCH 2 dans la gamme de concentration de 0.375% à 1.5% de sorte que cet extrait a une efficacité sur plusieurs représentants de la famille des protéines NOTCH.

### Exemple 2 - Compositions cosmétiques

L'extrait de menthe poivrée est utilisé à titre d'agent actif pour maintenir ou réguler le renouvellement des cellules de l'épiderme dans les compositions cosmétiques suivantes.

### 1 - Lotion

On prépare une solution aqueuse comprenant les actifs suivant (pourcentage en poids) :

|  | % |
|---|---|
| Calmiskin® | 2 |
| Glycérol | 3,0 |
| Extrait de Malva sylvestris | 0,2 |
| Hétérosides de Centella asiatica | 0,02 |
| Acides hyaluronique de haut et de bas poids moléculaire | 0,05 |
| Excipients | qsp 100 |

Calmiskin® est une solution d'extrait de menthe poivrée, commercialisée par la société SILAB, préparée à partir des parties aériennes de la plante.

La lotion est appliquée quotidiennement sur le visage.

2 - Crème de jour hydratante

On prépare une émulsion hydratante dont la formule est indiquée ci-dessous (% en poids)

|  | % |
|---|---|
| *Phase A* | |
| Calmiskin® | 0,75 |
| Solution à 1% en extrait de *Vanda coerulea* | 0,3 |
| Phénoxyéthanol | 0,5 |
| Gomme xanthane | 0,2 |
| Acrylates/C20-30 alkylacrylate crospolymères | 0,15 |
| EDTA tétrasodique | 0,1 |
| Eau | qsp |
| *Phase B* | |
| Polyisobutène hydrogéné | 4 |
| Squalane | 3 |
| Caprylique/caprique triglycéride | 3 |
| Pentylène glycol | 3 |
| Glycéryl stéarate | 3 |
| PEG-100 stéarate | 2,5 |
| Cire d'abeilles | 1,5 |
| Dicaprylyl carbonate | 1,5 |
| Cétyl alcool | 1 |
| Stéaryl alcool | 1 |
| Diméthicone | 1 |
| *Phase C* | |
| Hydroxyde de sodium | 0,04 |
| Eau | qsp 100 |

On disperse les gélifiants de la phase A dans l'eau puis on chauffe à 80-85°C, avant de solubiliser les composés. Les composés de la phase B sont chauffés à 85°C pour former une phase homogène. On émulsionne la phase A dans la phase B à l'aide d'un mélangeur Ystral.

L'émulsion huile/eau est finalement neutralisée à l'aide d'une solution aqueuse d'hydroxyde de sodium, puis refroidie.

La composition obtenue est une crème hydratante destinée à être appliquée sur le visage, en particulier sur les zones présentant des signes de vieillissement ou de sécheresse cutanée.

3- Crème de protection solaire de SPF 50

| INCI | % massique |
|---|---|
| Extrait aqueux de menthe poivrée | 0,1 |
| Eau | qs 100 |
| Butyleneglycol dicaprate/dicaprylate | 10,6 |
| Ethylhexyl methoxycinnamate | 7,5 |
| Methylene bis-benzotriazolyl tetramethylbutylphenol | 5 |
| Dicaprylyl carbonate | 4 |
| Butylene glycol | 3,7 |
| Bis-ethylhexyloxyphenol methoxyphenyl triazine | 3 |
| Dimethicone | 2 |
| Caprylyl methicone | 2 |

(suite)

| INCI | % massique |
|---|---|
| VP/Eicosene copolymer | 2 |
| Behenyl alcohol | 2 |
| Glycerol | 2 |
| Potassium cetyl phosphate | 2 |
| Cetearyl alcohol | 1,2 |
| phenylbenzimidazole sulfonic acid | 1 |
| tromethamine | 1 |
| Phenoxyethanol | 0,9 |

La crème présente un SPF d'une valeur égale à 50. La combinaison de l'extrait de menthe poivrée avec les filtres UV permet de protéger l'épiderme contre le rayonnement UV et permet de maintenir le renouvellement cellulaire cutané par kératinocytes progéniteurs.

## Revendications

1. Utilisation cosmétique d'une composition comprenant une quantité efficace d'un extrait de menthe poivrée, encore appelée *Mentha piperita,* pour traiter une peau sèche ou mature présentant des signes de vieillissement, de photo-vieillissement, de relâchement ou de sécheresse cutanée, **caractérisée en ce que** la composition stimule l'expression des protéines NOTCH au niveau des kératinocytes progéniteurs, et **en ce que** l'extrait de menthe poivrée est un extrait des parties aériennes obtenu par une hydrolyse enzymatique en milieu aqueux.

2. Utilisation selon la revendication 1, **caractérisée en ce que** les protéines NOTCH sont les protéines NOTCH 1 et NOTCH 2.

3. Utilisation selon la revendication 2, **caractérisée en ce que** la quantification de l'expression génique (RQ) du gène codant pour les protéines NOTCH 1 ou NOTCH 2 mesurée par PCR quantitative (qRT-PCR) en temps réel exprimée par rapport à un témoin non traité est supérieure à 1.

4. Utilisation selon l'une des revendications 1 à 3, **caractérisée en ce que** ladite composition contient de 0,005 à 0,5% en poids de matière sèche de l'extrait par rapport au poids de la composition.

5. Utilisation selon l'une des revendications 1 à 4, **caractérisée en ce que** la composition est appliquée sur le visage, et plus particulièrement sur le contour de l'œil, le décolleté, les lèvres et les mains, qui sont des zones exposées au rayonnement solaire.

6. Utilisation selon l'une des revendications 1 à 5, **caractérisée en ce que** la composition contient un actif agissant sur l'expression des protéines NOTCH choisi parmi un extrait aqueux de bourgeons de Hêtre (*Fagus Sylvatica*) ou un extrait aqueux de fleurs de *Bellis perennis.*

7. Utilisation selon l'une des revendications 1 à 6, **caractérisée en ce que** la composition contient un filtre UV et/ou des particules capables de réfléchir la lumière du soleil.

8. Méthode de soin cosmétique pour augmenter le renouvellement des cellules de l'épiderme d'une peau présentant des signes de vieillissement, de photo-vieillissement, de relâchement ou de sécheresse cutanée, ladite méthode comprenant l'application sur au moins une partie du visage ou du corps d'une quantité efficace d'un extrait de menthe poivrée, ledit extrait étant un extrait des parties aériennes obtenu par une hydrolyse enzymatique en milieu aqueux, qui stimule l'expression des protéines NOTCH au niveau des kératinocytes progéniteurs.

## Patentansprüche

1. Kosmetische Verwendung einer Zusammensetzung, die eine wirksame Menge eines Pfefferminzextrakts, auch *Mentha piperita* genannt, zur Behandlung von trockener Haut oder reifer Haut mit Anzeichen von Alterung, Lichtalterung, Absacken oder Hauttrockenheit umfasst, die **dadurch gekennzeichnet ist, dass** die Zusammensetzung die Expression von NOTCH Proteinen auf der Ebene von Vorläufer-Keratinozyten stimuliert, und **dadurch gekennzeichnet, dass** der Pfefferminz-Extrakt ein Extrakt der Luftteile ist, die durch enzymatische Hydrolyse in wässrigem Medium erhalten werden.

2. Verwendung nach Anspruch 1, **dadurch gekennzeichnet, dass** die NOTCH-Proteine NOTCH 1- und NOTCH 2-Proteine sind.

3. Verwendung nach Anspruch 2, **dadurch gekennzeichnet, dass** die Quantifizierung der Genexpression (RQ) des Gens, das die NOTCH 1- oder NOTCH 2-Proteinen codiert, gemessen durch quantitative PCR (qRT-PCR) in Echtzeit, ausgedrückt relativ zu einer unbehandelten Kontrolle, ist größer als 1.

4. Verwendung nach einem der Ansprüche 1 bis 3, **dadurch gekennzeichnet, dass** die Zusammensetzung 0,005 bis 0,5 Gew .-% Trockenmasse des Extrakts bezogen auf das Gewicht der Zusammensetzung enthält.

5. Verwendung gemäß einem der Ansprüche 1 bis 4, **dadurch gekennzeichnet, dass** die Zusammensetzung auf das Gesicht und insbesondere auf die Kontur des Auges, den Ausschnitt, die Lippen und die Hände aufgetragen wird, die Bereiche der Sonne Strahlung ausgesetzt sind.

6. Verwendung nach einem der Ansprüche 1 bis 5, **dadurch gekennzeichnet, dass** die Zusammensetzung einen Wirkstoff enthält, der auf die Expression von NOTCH-Proteinen wirkt, und der aus einem wässrigen Extrakt von Buchenknospen (*Fagus Sylvatica*) oder einem wässrigen Extrakt von Buchenblüten *Bellis perennis* ausgewählt ist.

7. Verwendung nach einem der Ansprüche 1 bis 6, **dadurch gekennzeichnet, dass** die Zusammensetzung einen UV-Filter und / oder Partikel enthält, die Sonnenlicht reflektieren können.

8. Verfahren zur kosmetischen Hautpflege zur Erhöhung der Erneuerung von Zellen der Epidermis einer Haut, die Anzeichen von Alterung, Lichtalterung, Absacken oder Hauttrockenheit zeigen, wobei das Verfahren das Auftragen auf mindestens einen Teil des Gesichts oder des Körpers wirksame Menge eines Pfefferminz-Extrakts umfasst, wobei der Extrakt ein Extrakt der Luftteile ist, der durch enzymatische Hydrolyse in einem wässrigen Medium erhalten wird und die Expression von NOTCH-Proteinen auf der Ebene von Vorläufer-Keratinozyten stimuliert.

## Claims

1. Cosmetic use of a composition comprising an effective amount of a peppermint extract, also called *Mentha piperita,* for treating dry skin or mature skin exhibiting signs of aging, photoaging, sagging or cutaneous dryness, **characterized in that** the composition stimulates the expression of NOTCH proteins at the level of progenitor keratinocytes, and **characterized in that** said peppermint extract is an extract of the aerial parts obtained by enzymatic hydrolysis in aqueous medium.

2. Use according to claim 1, **characterized in that** the NOTCH proteins are the NOTCH 1 and NOTCH 2 proteins.

3. Use according to claim 2, **characterized in that** the quantification of the gene expression (RQ) of the gene encoding the NOTCH 1 or NOTCH 2 proteins measured by quantitative PCR (qRT-PCR) in real time expressed relative to a untreated control is greater than 1.

4. Use according to one of claims 1 to 3, **characterized in that** said composition contains 0.005 to 0.5% by weight of dry matter of the extract relative to the weight of the composition.

5. Use according to one of claims 1 to 4, **characterized in that** the composition is applied to the face, and more particularly to the contour of the eye, the neckline, the lips and the hands, which are areas that are exposed to solar radiation.

6. Use according to one of claims 1 to 5, **characterized in that** the composition contains an active agent acting on the expression of NOTCH proteins chosen from an aqueous extract of beech buds (*Fagus Sylvatica*) or an aqueous extract of beech flowers *Bellis perennis.*

7. Use according to one of claims 1 to 6, **characterized in that** the composition contains a UV filter and/or particles capable of reflecting sunlight.

8. A method of cosmetic skin care for increasing the renewal of cells of the epidermis of a skin showing signs of aging, photoaging, sagging or cutaneous dryness, said method comprising application to at least a part of the face or of the body of an effective amount of an extract of peppermint, said extract being an extract of the aerial parts obtained by enzymatic hydrolysis in an aqueous medium, that stimulates the expression of NOTCH proteins at the level of progenitor keratinocytes.

**FIGURE 1**

**FIGURE 2**

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

## Documents brevets cités dans la description

- FR 2848851
- FR 2895678
- JP 2007277149 A
- JP 2006176436 A
- JP 2012236801 A
- FR 2962648 A1
- FR 2978043 A1
- FR 2848851 A1
- FR 2895678 A1
- EP 669323 A
- US 2463264 A

## Littérature non-brevet citée dans la description

- **AITHAL M. G. S et al.** Role of NOTCH signaling pathway in cancer and its association with DNA methylation. *J. Genet.,* 2013
- **MELNIK BC.** The Potential Role of Impaired NOTCH Signalling in Atopic Dermatitis. *Acta Derm Venereol.,* 2014
- **MELNIK BC.** The Potential Role of Impaired NOTCH Signalling in Atopic Dermatitis. *Acta Derm Venereol,* 2014
- **SELEIT I et al.** Immunohistochemical expression of aberrant NOTCH -1 signaling in vitiligo: an implication for pathogenesis. *Ann Diagn Pathol.,* 2014
- **NOWELL C.** Cutaneous NOTCH signaling in health and disease. *Cold Spring Harb Perspect Med.,* 2013
- **AITHAL M. G. S et al.** Role of NOTCH signalling pathway in cancer and its association with DNA methylation. *J. Genet,* 2013
- **LIRSTEN A.** Cutaneous wound healing: recruiting developmental pathways for regeneration. *Cell. Mol. Life Sci.,* 2013
- **PALAZZO E et al.** NOTCH-1 and NOTCH-2 modulate keratinocyte stem cell viability and differentiation during skin ageing and UVB exposure. *J Invest Dermatol,* 2011, vol. 131, S116-S117
- **PALAZZO P et al.** S2 NOTCH 1 downregulates human keratinocyte differentiation: a feedback loop with survivin. *J Invest Dermatol,* 2014
- Skin Aquaporins: Function in Hydration, Wound Healing, and Skin Epidermis Homeostasis. **M BOURY-JAMOT et al.** Aquaporins, Handbook of Experimental Pharmacology 190. Springer-Veriag Berlin Heidelberg, 2009, 205-217
- **MARCONI A et al.** Expression and Function of Neurotrophins and Their Receptors in Cultured Human Keratinocytes. *J Invest Dermatol,* 2003, vol. 121, 1515-1521
- **PALAZZO E et al.** Role of neurotrophins on dermal fibroblast survival and differentiation. *J Cell Physiol.,* 2012, vol. 227, 1017-25
- **PROVOST N et al.** Ultraviolet A radiation transiently disrupts gap junctional communication in human keratinocytes. *Am J Physiol Cell Physiol,* 2003, vol. 284, C51-C59
- **BARRANDON Y ; GREEN H.** *Proc Natl Acad Sci USA,* 1987, vol. 84, 2302-2306